(19) Europäisches Patentamt
European Patent Office
Office européen des brevets

(11) EP 0 589 874 B1

(12) # EUROPÄISCHE PATENTSCHRIFT

(45) Veröffentlichungstag und Bekanntmachung des Hinweises auf die Patenterteilung:
**08.09.1999 Patentblatt 1999/36**

(21) Anmeldenummer: **91911959.4**

(22) Anmeldetag: **21.06.1991**

(51) Int Cl.[6]: **C07D 295/12**, A61K 31/445

(86) Internationale Anmeldenummer:
**PCT/EP91/01147**

(87) Internationale Veröffentlichungsnummer:
**WO 93/00337 (07.01.1993 Gazette 1993/02)**

(54) **VERWENDUNG VON (S)(+)-2-ÄTHOXY-4-[N-[1-(2-PIPERIDINO-PHENYL)-3-METHYL-1-BUTYL]AMINOCARBONYLMETHYL]-BENZOESÄURE ZUR HERSTELLUNG EINES LANGZEITANTIDIABETIKUMS**

USE OF (S)(+)-2-ETHOXY-4-[N-[1-(2-PIPERIDINOPHENYL)-3-METHYL-1-BUTYL]AMINOCARBONYLMETHYL]BENZOIC ACID FOR THE PREPARATION OF A LONGLASTING ANTIDIABETIC MEDICAMENT

UTILISATION DE L'ACIDE (S)(+)-2-ETHOXY-4-[N-[1-(2-PIPERIDINO-PHENYL)-3-METHYL-1-BUTYL]AMINOCARBONYLMETHYL]-BENZOIQUE POUR LA PREPARATION D'UN MEDICAMENT ANTIDIABETIQUE DE LONGUE DUREE

(84) Benannte Vertragsstaaten:
**AT BE CH DE DK ES FR GB GR IT LI LU NL SE**

(43) Veröffentlichungstag der Anmeldung:
**06.04.1994 Patentblatt 1994/14**

(60) Teilanmeldung: **99101810.2**

(73) Patentinhaber: **Boehringer Ingelheim Pharma KG
55216 Ingelheim am Rhein (DE)**

(72) Erfinder:
- **GRELL, Wolfgang
D-7950 Biberach 1 (DE)**
- **GREISCHEL, Andreas
D-55411 Bingenh 1 (DE)**
- **ZAHN, Gabriele
CH-8006 Zürich (CH)**
- **MARK, Michael
D-7950 Biberach (DE)**
- **KNORR, Hansjörg
D-6507 Ingelheim (DE)**
- **RUPPRECHT, Eckhard
A-2340 Mödling (AT)**
- **MÜLLER, Ülrich
D-7950 Biberach (DE)**

(74) Vertreter: **Laudien, Dieter, Dr.
Boehringer Ingelheim GmbH,
Binger Strasse 173
55216 Ingelheim (DE)**

(56) Entgegenhaltungen:
**EP-A- 0 207 331**

- **JOURNAL OF CHROMATOGRAPHY, BIOMEDICAL APPLICATIONS. Bd. 568, Nr. 1, AMSTERDAM NL Seiten 246 - 252; Greischel A; Beschke K; Rapp H; Roth W: 'Quantitation of the new hypoglycemic agent AG-EE 388 ZW in human plasma by automated high-performance liquid chromatography with electrochemical detection' SA 48558 030siehe Seite 252, Absatz 3**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 589 874 B1

Printed by Jouve, 75001 PARIS (FR)

## Beschreibung

In der EP-B-0,147,850 wird unter anderem das Racemat von 2-Äthoxy-4-[N-[1-(2-piperidino-phenyl)-3-methyl-1-butyl]aminocarbonylmethyl]-benzoesäure (Code-Nr.: AG-EE 388 ZW) der Formel

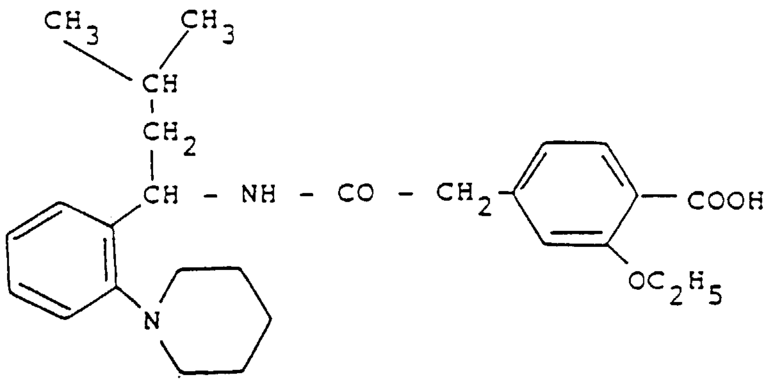

und in der EP-B-0,207,331 werden zwei weitere polymorphe Formen dieser Verbindung beschrieben. Diese Verbindung und deren physiologisch verträgliche Salze weisen wertvolle pharmakologische Eigenschaften auf, nämlich eine Wirkung auf den Intermediärstoffwechsel, insbesondere jedoch eine blutzuckersenkende Wirkung.

Die beiden Enantiomeren dieser Verbindung, nämlich (S)(+)-2-Äthoxy-4-[N-[1-(2-piperidino-phenyl)-3-methyl-1-butyl] aminocarbonylmethyl]-benzoesäure (Code-Nr.: AG-EE 623 ZW) und (R)(-)-2-Äthoxy-4-[N-[1-(2-piperidino-phenyl)-3-methyl-1-butyl]aminocarbonylmethyl]-benzoesäure (Code-Nr.: AG-EE 624 ZW), wurden auf ihre blutzuckersenkende Wirkung an weiblichen Ratten geprüft.

Überraschenderweise wurde gefunden, daß das (S)-Enantiomere (AG-EE 623 ZW) das wirksame Enantiomere ist, und daß seine Wirkung länger als 6 Stunden an der Ratte anhält.

Auf Grund dieser Ergebnisse an der Ratte erscheint für den Menschen die ausschließliche Verwendung von AG-EE 623 ZW geboten, wodurch die Dosis im Vergleich zur AG-EE 388 ZW-Dosis um 50 % reduziert werden kann. Dies und eine relativ lange Wirkungsdauer konnten am Menschen bestätigt werden. Darüber hinaus wurde jedoch bei den Human-Studien gefunden, daß AG-EE 623 ZW überraschende pharmakokinetische Eigenschaften aufweist, die auf Grund der AG-EE 388 ZW-Daten nicht vorhersehbar waren. AG-EE 623 ZW zeigt somit überraschende therapeutische Vorteile gegenüber dem Racemat AG-EE 388 ZW.

Die überraschenden Befunde am Menschen sind:

(a) Die AG-EE 623 ZW-Spiegel fallen rascher gegen null als die AG-EE 388 ZW-Spiegel, selbst bei gleicher absoluter Dosis, was aufgrund der relativ langen Wirkungsdauer nicht zu erwarten war.
(b) In Relation zur erzielten Blutzuckersenkung treten wesentlich niedrigere Plasmaspiegel von AG-EE 623 ZW auf, als bei einer Halbierung der AG-EE 388 ZW-Dosis zu erwarten gewesen wären,
(c) Die blutzuckersenkende Wirkung tritt nach AG-EE 623 ZW-Gabe rascher ein als nach AG-EE 388 ZW-Gabe.

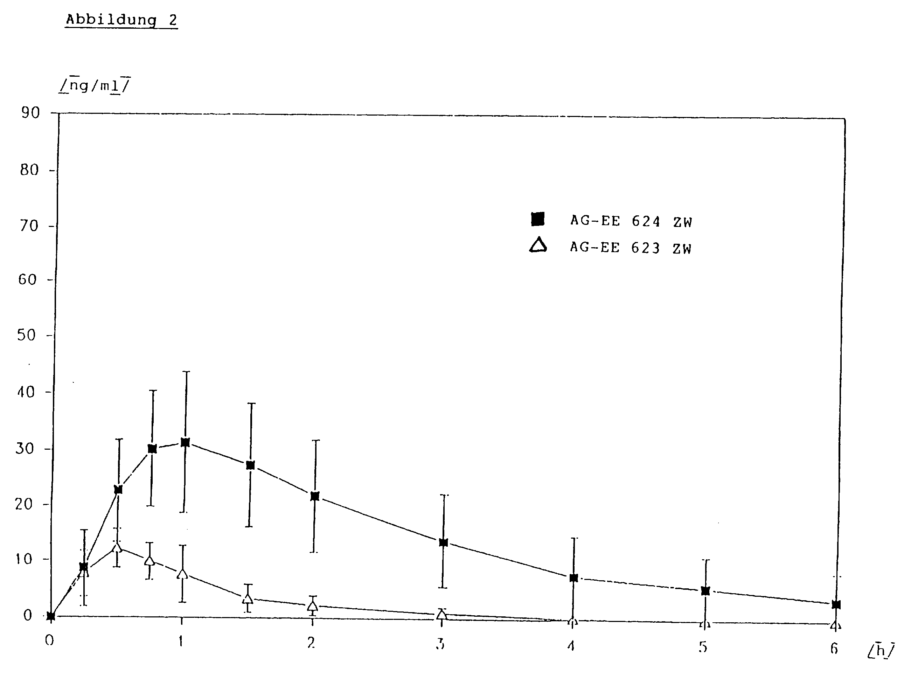

Der eklatante Unterschied zwischen den beiden Enantiomeren besteht darin, daß das wirksame Enantiomere, AG-EE 623 ZW, überraschenderweise trotz relativ langer Wirkdauer wesentlich schneller als das unwirksame Enantiomere, AG-EE 624 ZW, eliminiert wird, wie die Abbildungen 1 und 2 demonstrieren. Abbildung 1 gibt hierbei die Plasma-Konzentration von AG-EE 623 ZW und AG-EE 624 ZW nach 1,0 mg i.v. AG-EE 388 ZW und Abbildung 2 die Plasma-Konzentration von AG-EE 623 ZW und AG-EE 624 ZW nach 1,0 mg p.o. (Lösung) AG-EE 388 ZW jeweils an 12 freiwilligen männlichen Probanden wieder. Nach Racemat-Gabe ist das unwirksame Enantiomere, AG-EE 624 ZW, also nicht nur als unnötiger Ballast in gleich hohen Plasma-Konzentrationen wie das wirksame Enantiomere, AG-EE 623 ZW, sondern in unerwartet höheren Maximal- und Dauer-Spiegeln anwesend. Dies wirkt sich z.B. bei Applikation einer Tablette mit 2 mg AG-EE 388 ZW bzw. einer Tablette mit 1 mg AG-EE 623 ZW an 12 bzw. 6 Probanden so aus, daß die Maximal-Konzentrationen 84 ± 25 bzw. 28 ± 18 ng/ml sowie die Konzentrationen nach 4 Stunden 19 ± 8 bzw. 0,7 ± 1,0 ng/ml, nach 5 Stunden 13 ± 6 bzw. 0,3 ± 0,7 ng/ml und nach 6 Stunden 10 ± 6 bzw. 0,3 ± 0,7 ng/ml betragen.

Das überraschend schnelle Einsetzen der Blutzuckersenkung von AG-EE 623 ZW im Vergleich zu AG-EE 388 ZW ist für Diabetiker besonders vorteilhaft, weil das schnelle Einsetzen eine optimale Kontrolle der Krankheit zur Folge hat.

Gegenüber der AG-EE 388 ZW-Applikation besteht also der überraschende Vorteil der AG-EE 623 ZW-Applikation darin, daß unnötig hohe und langdauernde Substanz-Belastungen des Organismus vermieden werden, was

bei einer Langzeit-Therapie wie der des Diabetes Mellitus von großer Bedeutung ist.

Aus den Human-Studien geht hervor, daß das (S)-Enantiomere, nämlich (S)(+)-2-Äthoxy-4-[N-[1-(2-piperidino-phenyl)-3-methyl-1-butyl]aminocarbonylmethyl]-benzoe-säure, als Träger der blutzuckersenkenden Wirksamkeit infolge seiner überraschend und auf Grund der relativ langen Wirkdauer nicht vorhersehbar schnellen Elimination aus dem Blut im Vergleich mit AG-EE 388 ZW überlegene Eigenschaften aufweist, die weit über den "normalen" Vorteil eines Enantiomers gegenüber seinem Racemat, nämlich den Vorteil der Dosis-Halbierung, hinausgehen.

Gegenstand der vorliegenden Erfindung ist also die Verwendung von (S)(+)-2-Äthoxy-4-[N-[1-(2-piperidino-phenyl)-3-methyl-1-butyl]aminocarbonylmethyl]-benzoesäure beispielsweise mit einer optische Reinheit von mindestens ee = 95 %, vorzugsweise von mindestens 98 %, deren physiologisch verträgliche Salze mit anorganischen oder organischen Säuren oder Basen, zur Herstellung eines Langzeitantidiabeticums, das im Vergleich zu der doppelten Einzeldosis bei einer Racemat-Applikation unnötig hohe und langandauernde Substanzbelastungen vermeidet, wodurch wesentlich niedrigere Wirkstoff-Plasmaspiegel auftreten, die über den normalen Vorteil der Dosis-Halbierung bei der Enantiomeren-Applikation hinausgehen.

Zweckmäßigerweise erhält man den Wirkstoff nach folgenden Verfahren:

a) Umsetzung des (S)-Amins der Formel

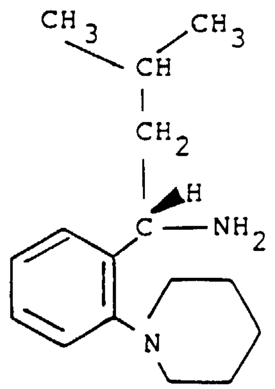

,(I)

mit einer Carbonsäure der allgemeinen Formel

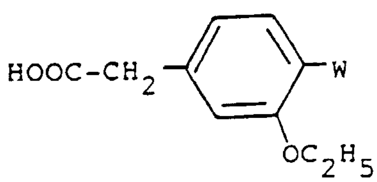

,(II)

in der

W eine Carboxygruppe oder eine durch einen Schutzrest geschützte Carboxygruppe darstellt,

oder mit deren gegebenenfalls im Reaktionsgemisch hergestellten reaktionsfähigen Derivaten und erforderlichenfalls anschließende Abspaltung eines Schutzrestes.

Als reaktionsfähige Derivate einer Verbindung der allgemeinen Formel II kommen beispielsweise deren Ester wie der Methyl-, Äthyl- oder Benzylester, deren Thioester wie der Methylthio- oder Äthylthioester, deren Halogenide wie das Säurechlorid, deren Anhydride oder Imidazolide in Betracht.

Die Umsetzung wird zweckmäßigerweise in einem Lösungsmittel wie Methylenchlorid, Chloroform, Tetrachlorkohlenstoff, Äther, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureäthylester, Chlorameisensäureisobutylester, Thionylchlorid, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiären organischen Base wie Triäthylamin oder Pyridin, welche gleichzeitig als Lösungsmittel dienen können, bei Temperaturen zwischen -25 und 250°C, vorzugsweise jedoch bei Temperaturen zwischen -10°C und der Siedetemperatur des verwendeten Lösungsmittels, durchgeführt. Die Umsetzung kann

auch ohne Lösungsmittel durchgeführt werden, desweiteren kann während der Umsetzung entstehendes Wasser durch azeotrope Destillation, z.B. durch Erhitzen mit Toluol am Wasserabscheider, oder durch Zugabe eines Trokkenmittels wie Magnesiumsulfat oder Molekularsieb abgetrennt werden.

Erforderlichenfalls wird die anschließende Abspaltung eines Schutzrestes vorzugsweise hydrolytisch durchgeführt, zweckmäßigerweise entweder in Gegenwart einer Säure wie Salzsäure, Schwefelsäure, Phosphorsäure, Trifluoressigsäure oder Trichloressigsäure oder in Gegenwart einer Base wie Natriumhydroxid oder Kaliumhydroxid in einem geeigneten Lösungsmittel wie Wasser, Methanol, Methanol/Wasser, Äthanol, Äthanol/Wasser, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches.

Ein als Schutzrest verwendeter tert.Butylrest kann auch thermisch gegebenenfalls in einem inerten Lösungsmittel wie Methylenchlorid, Chloroform, Benzol, Toluol, Tetrahydrofuran, Dioxan oder Eisessig und vorzugsweise in Gegenwart einer starken Säure wie Trifluoressigsäure, Bromwasserstoff, p-Toluolsulfonsäure, Schwefelsäure, Phosphorsäure oder Polyphosphorsäure abgespalten werden.

Desweiteren kann ein als Schutzrest verwendeter Benzylrest auch hydrogenolytisch in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid abgespalten werden.

b) Spaltung einer (S)-Verbindung der allgemeinen Formel

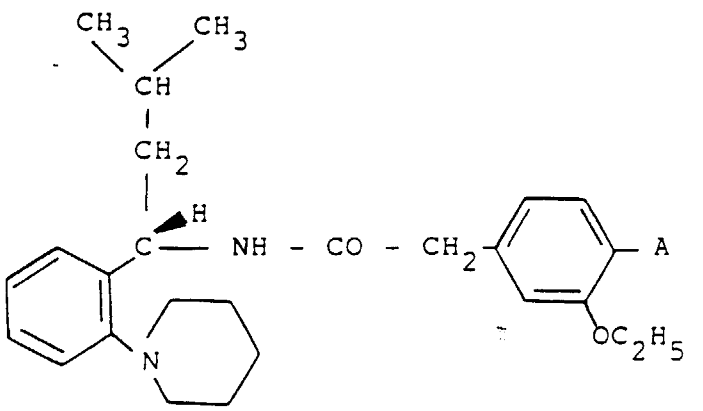

,(III)

in der

A eine durch Hydrolyse, Thermolyse oder Hydrogenolyse in eine Carboxygruppe überführbare Gruppe darstellt.

Als hydrolysierbare Gruppen kommen beispielsweise funktionelle Derivate der Carboxygruppe wie deren unsubstituierte oder substituierte Amide, Ester, Thioester, Orthoester, Iminoäther, Amidine oder Anhydride, die Nitrilgruppe, die Tetrazolylgruppe, eine gegebenenfalls substituierte 1,3-Oxazol-2-yl- oder 1,3-Oxazolin-2-yl-gruppe und

als thermolytisch abspaltbare Gruppen beispielsweise Ester mit tertiären Alkoholen, z.B. der tert.Butylester, und als hydrogenolytisch abspaltbare Gruppen beispielsweise Aralkylgruppen, z.B. die Benzylgruppe, in Betracht.

Die Hydrolyse wird zweckmäßigerweise entweder in Gegenwart einer Säure wie Salzsäure, Schwefelsäure, Phosphorsäure, Trifluoressigsäure oder Trichloressigsäure oder in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches, durchgeführt.

Bedeutet A in einer Verbindung der allgemeinen Formel III eine Nitril- oder Aminocarbonylgruppe, so können diese Gruppen mittels 100%iger Phosphorsäure bei Temperaturen zwischen 100 und 180°C, vorzugsweise bei Temperaturen zwischen 120 und 160°C, oder auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen 0 und 50°C in die Carboxygruppe übergeführt werden.

Bedeutet A in einer Verbindung der allgemeinen Formel III beispielsweise die tert.Butyloxycarbonylgruppe, so kann die tert.Butylgruppe auch thermisch gegebenenfalls in einem inerten Lösungmittel wie Methylenchlorid, Chloroform, Benzol, Toluol, Tetrahydrofuran, Dioxan oder Eisessig und vorzugsweise in Gegenwart einer starken Säure wie Trifluoressigsäure, Bromwasserstoff, p-Toluolsulfonsäure, Schwefelsäure, Phosphorsäure oder Polyphosphorsäure bei Temperaturen zwischen 0 und 100°C, vorzugsweise bei Temperaturen zwischen 20°C und der

Siedetemperatur des verwendeten Lösungsmittel, abgespalten werden.

Bedeutet A in einer Verbindung der allgemeinen Formel III beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Methanol/Wasser, Äthanol/Wasser, Eisessig, Essigsäureäthylester, Dioxan oder Dimethylformamid vorzugsweise bei Temperaturen zwischen 0 und 50°C, z.B. bei Raumtemperatur und einem Wasserstoffdruck von 1 bis 5 bar abgespalten werden.

c) Umsetzung einer (S)-Verbindung der allgemeinen Formel

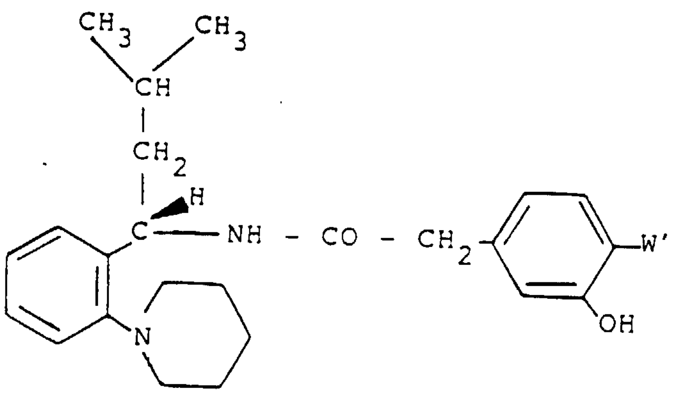

, (IV)

in der

W' eine Carboxygruppe oder eine Alkoxycarbonylgruppe mit insgesamt 2 bis 5 Kohlenstoffatomen, wobei der Alkylteil der Alkoxygruppe durch eine Phenylgruppe substituiert sein kann, bedeutet,

mit einer Verbindung der allgemeinen Formel

$$Z - CH_2 - CH_3, \qquad (V)$$

in der

Z eine nukleophil austauschbare Gruppe wie ein Halogenatom, eine Sulfonyloxygruppe oder auch zusammen mit dem benachbarten Wasserstoffatom eine Diazogruppe darstellt und erforderlichenfalls anschließende Hydrolyse oder Hydrogenolyse.

Die Umsetzung wird zweckmäßigerweise mit einem entsprechenden Halogenid, Sulfonsäureester oder Schwefelsäurediester, z.B. mit Äthylbromid, Äthyljodid, Diäthylsulfat, p-Toluolsulfonsäure-äthylester oder Methansulfonsäure-äthylester, oder mit Diazoäthan, gegebenenfalls in Gegenwart einer Base wie Natriumhydrid, Kaliumcarbonat, Natriumhydroxid, Kalium-tert.butylat oder Triäthylamin vorzugsweise in einem geeigneten Lösungsmittel wie Aceton, Diäthyläther, Tetrahydrofuran, Dioxan, Pyridin oder Dimethylformamid bei Temperaturen zwischen 0 und 100°C, vorzugsweise bei Temperaturen zwischen 20 und 50°C, durchgeführt.

Bedeutet W' in einer Verbindung der allgemeinen Formel IV eine Carboxygruppe, so kann diese in die entsprechende Esterverbindung übergeführt werden.

Erforderlichenfalls wird die anschließende Hydrolyse entweder in Gegenwart einer Säure wie Salzsäure, Schwefelsäure, Phosphorsäure, Trifluoressigsäure oder Trichloressigsäure oder in Gegenwart einer Base wie Natriumhydroxid oder Kaliumhydroxid in einem geeigneten Lösungsmittel wie Wasser, Methanol, Methanol/Wasser, Äthanol, Äthanol/Wasser, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches, oder die anschließende Hydrogenolyse in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid bei einem Wasserstoffdruck von 1 bis 10 bar durchgeführt.

d) Enantioselektive Reduktion einer Verbindung der allgemeinen Formel

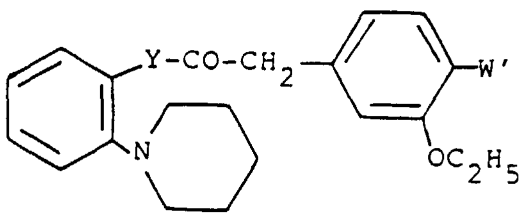

,(VI)

in der

W' eine Carboxygruppe oder eine Alkoxycarbonylgruppe mit insgesamt 2 bis 5 Kohlenstoffatomen, wobei der Alkylteil der Alkoxygruppe durch eine Phenylgruppe substituiert sein kann, und

Y eine Gruppe der Formel

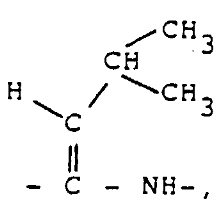

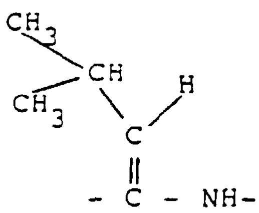

oder

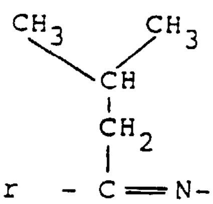

darstellt und erforderlichenfalls anschließende Hydrolyse.

Die Reduktion wird vorzugsweise mit Wasserstoff in Gegenwart eines geeigneten chiralen Hydrierungskatalysators in einem geeigneten Lösungsmittel wie Methanol, Äthanol, Isopropanol, Äthylacetat, Dioxan, Tetrahydrofuran, Methanol/Tetrahydrofuran, Methanol/Methylenchlorid, Äthanol/Methylenchlorid oder Isopropanol/Methylenchlorid bei Temperaturen zwischen 0 und 100°C, vorzugsweise jedoch bei Temperaturen zwischen 20 und 50°C, und einem Wasserstoffdruck zwischen 1 und 1 000 bar, vorzugsweise zwischen 5 und 100 bar, und zweckmäßigerweise unter Zusatz von 0,1 bis 5 %, vorzugsweise 0,3 bis 1 %, Titan(IV)tetraisopropylat, und vorzugsweise unter Ausschluß von Luftsauerstoff ausgeführt. Vorzugsweise wird die Reduktion mit der (Z)-Form einer Verbindung der allgemeinen Formel VI durchgeführt.

Als chirale Hydrierungskatalysatoren kommen entsprechende Metalligandenkomplexe wie $Ru(OCO\text{-}CH_3)_2$ [(S)-BINAP], $Ru_2Cl_4$[(S)-BINAP]$_2$ x $N(C_2H_5)_3$, Rh[(S)-BINAP-NBD]$ClO_4$ oder Rh[(-)-NORPHOS-COD]$BF_4$ in Betracht.

Bei der katalytischen Hydrierung kann eine Benzyloxycarbonylgruppe gleichzeitig mitreduziert und in die Carboxygruppe überführt werden.

Erforderlichenfalls wird die anschließende Hydrolyse entweder in Gegenwart einer Säure wie Salzsäure, Schwefelsäure, Phosphorsäure, Trifluoressigsäure oder Trichloressigsäure oder in Gegenwart einer Base wie Natriumhydroxid oder Kaliumhydroxid in einem geeigneten Lösungsmittel wie Wasser, Methanol, Methanol/Wasser, Äthanol, Äthanol/Wasser, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches, durchgeführt.

e) Oxidation einer (S)-Verbindung der allgemeinen Formel

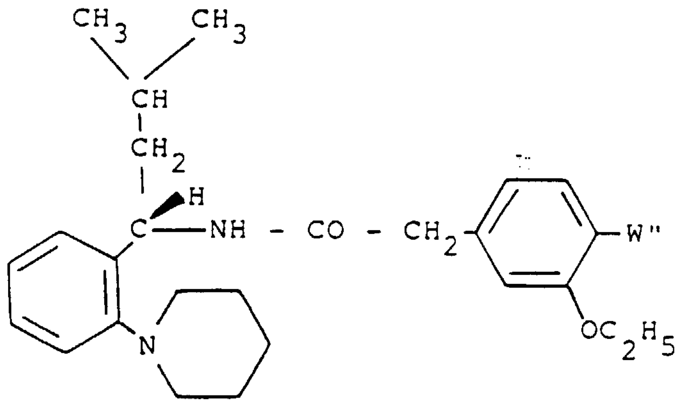

,(VII)

in der
W" eine durch Oxidation in eine Carboxygruppe überführbare Gruppe darstellt.

Als eine derartige oxidierbare Gruppe kommt beispielsweise die Formylgruppe und deren Acetale, die Hydroxymethylgruppe und deren Äther, eine unsubstituierte oder substituierte Acylgruppe wie die Acetyl-, Chloracetyl-, Propionyl-, die Malonsäure-(1)-yl-gruppe oder eine Malonester-(1)-yl-gruppe in Betracht.

Die Umsetzung wird mit einem Oxidationsmittel in einem geeigneten Lösungsmittel wie Wasser, Eisessig, Methylenchlorid, Dioxan oder Glykoldimethyläther bei Temperaturen zwischen 0 und 100°C, zweckmäßigerweise jedoch bei Temperaturen zwischen 20°C und 50°C, durchgeführt. Die Umsetzung wird jedoch vorzugsweise mit Silberoxid/Natronlauge, Mangandioxid/Aceton oder Methylenchlorid, Wasserstoffperoxid/Natronlauge, Brom oder Chlor/Natron- oder Kalilauge, Chromtrioxid/Pyridin oder Pyridiniumchlorchromat durchgeführt.

f) Auftrennung eines Gemisches, bestehend aus einer beliebigen Menge des (S)-Enantiomeren der allgemeinen Formel

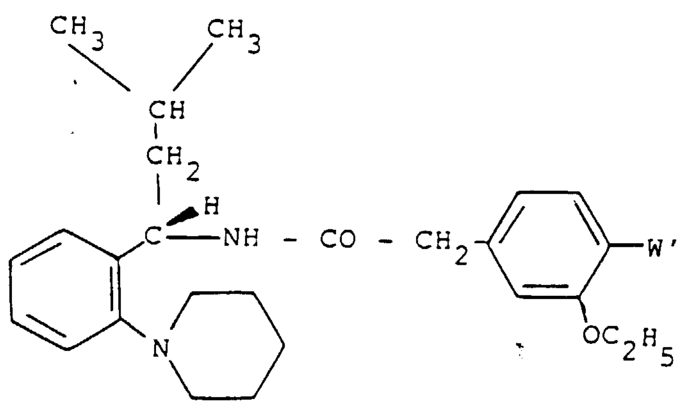

,(VIII)

und einer beliebigen Menge des (R)-Enantiomeren der allgemeinen Formel

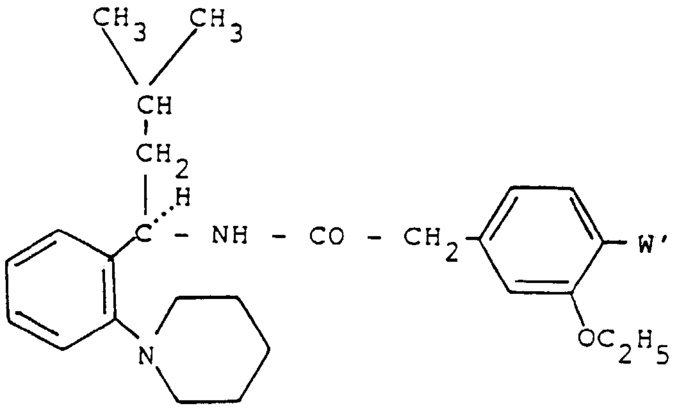

,(IX)

in denen

W' eine Carboxygruppe oder eine Alkoxycarbonylgruppe mit insgesamt 2 bis 5 Kohlenstoffatomen, wobei der Alkylteil der Alkoxygruppe durch eine Phenylgruppe substituiert sein kann, bedeutet, vorzugsweise eines 50/50-Gemisches, über deren diastereomeren Addukte, Komplexe oder Salze und erforderlichenfalls anschließende Hydrolyse oder Hydrogenolyse.

Die Auftrennung wird vorzugsweise mittels Säulen- oder HPL-Chromatographie durch Bildung der diastereomeren Addukte oder Komplexe an einer chiralen Phase durchgeführt.

Erforderlichenfalls wird die anschließende Hydrolyse entweder in Gegenwart einer Säure wie Salzsäure, Schwefelsäure, Phosphorsäure, Trifluoressigsäure oder Trichloressigsäure oder in Gegenwart einer Base wie Natriumhydroxid oder Kaliumhydroxid in einem geeigneten Lösungsmittel wie Wasser, Methanol, Methanol/Wasser, Äthanol, Äthanol/Wasser, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches, oder

die anschließende Hydrogenolyse in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid bei einem Wasserstoffdruck von 1 bis 10 bar durchgeführt.

Das so erhaltene (S)-Enantiomere mit einer optischen Reinheit von zweckmäßigerweise mindestens 90 % läßt sich durch fraktionierte Kristallisation in ein <5)-Enantiomer von mindestens 95 %, vorzugsweise 98 bis 100 % optischer Reinheit, überführen.

Das gleiche gilt für die (S)-Verbindungen der Formeln III, IV und VII, insbesondere für deren Ester.

Das so erhaltene (S)-Enantiomere läßt sich in seine Salze, insbesondere zur pharmazeutischen Anwendung in seine physiologisch verträglichen Salze mit anorganischen oder organischen Säuren oder auch Basen überführen. Als Säuren kommen hierbei beispielsweise Salzsäure, Bromwasserstoffsäure, Schwefelsäure, Phosphorsäure, Milchsäure, Zitronensäure, Weinsäure, Bernsteinsäure, Maleinsäure oder Fumarsäure und als Basen Natriumhydroxid, Kaliumhydroxid, Kalziumhydroxid, Cyclohexylamin, Äthanolamin, Diäthanolamin, Triäthanolamin, Äthylendiamin oder Lysin in Betracht.

Die als Ausgangsstoffe verwendeten Verbindungen der Formeln I bis IX sind teilweise literaturbekannt bzw. erhält man nach an sich bekannten Verfahren.

Das (S)-Amin der Formel I kann aus dem entsprechenden racemischen Amin

durch Racematspaltung, z.B. mittels fraktionierter Kristallisation der diastereomeren Salze mit geeigneten optisch aktiven Säuren, vorzugsweise mit N-Acetyl-L-glutaminsäure, und erforderlichenfalls Umkristallisation sowie anschließender Zerlegung der Salze,

durch Säulen- oder HPL-Chromatographie an chiralen Phasen, gegebenenfalls in Form eines Acylderivates,

oder durch Bildung von diastereomeren Verbindungen, deren Trennung und anschließende Spaltung erhalten werden.

Ferner kann das (S)-Amin der Formel I

durch enantioselektive Reduktion mittels Wasserstoff in Gegenwart eines geeigneten chiralen Hydrierungskatalysators ausgehend von einem entsprechenden N-Acyl-ketimin bzw. Enamid, zweckmäßigerweise unter Zusatz von 0,1 bis 5 % Titantetraisopropylat, und gegebenenfalls anschließende Abspaltung des Acylrestes wie des Formyl- oder Acetylrestes,

durch diastereoselektive Reduktion eines entsprechenden am Stickstoffatom chiral substituierten Ketimins oder Hydrazins mittels Wasserstoff in Gegenwart eines geeigneten Hydrierungskatalysators, zweckmäßigerweise unter Zusatz von 0,1 bis 5 % Titantetraisopropylat, und gegebenenfalls anschließende Abspaltung des chiralen Hilfsrestes, z.B. des (S)-1-Phenäthyl-Restes durch katalytische Hydrogenolyse, oder

durch diastereoselektive Addition einer entsprechenden metallorganischen Verbindung, vorzugsweise einer Grignard- oder einer Lithiumverbindung, an ein entsprechendes am Stickstoffatom chiral substituiertes Aldimin, gegebenenfalls unter Zusatz von 0,1 bis 10 % Titantetraisopropylat, anschließende Hydrolyse und gegebenenfalls Trennung der erhaltenen Diastereomeren und anschließende Abspaltung des chiralen Hilfsrestes, z.B. des (R)-1-Phenäthyl-Restes durch katalytische Hydrogenolyse,
hergestellt und erforderlichenfalls durch Salzbildung mit geeigneten optisch aktiven Säuren, vorzugsweise mit N-Acetyl-L-glutaminsäure, und erforderlichenfalls ein- oder mehrfache Umkristallisation sowie anschließende Zerlegung des Salzes in hoher Enantiomeren-Reinheit erhalten werden.

Die als Ausgangsstoffe verwendeten Verbindungen der allgemeinen Formeln III, IV und VII erhält man durch Umsetzung des (S)-Amins I mit einer entsprechenden Carbonsäure bzw. deren reaktiven Derivaten und gegebenenfalls anschließende Abspaltung eines verwendeten Schutzrestes.

Die als Ausgangsstoff verwendete Verbindung der allgemeinen Formel VI erhält man durch Acylierung der entsprechenden Iminoverbindung oder deren metallorganischen Komplexen mit der entsprechenden Carbonsäure oder mit deren reaktiven Derivaten mit gegebenenfalls anschließender Spaltung einer Estergruppe.

Das (S)-Enantiomere ist praktisch untoxisch; beispielsweise verstarb nach einer einmaligen Applikation von 1000 mg/kg p.o. (Suspension in 1%iger Methylcellulose) an jeweils 5 männliche und 5 weibliche Ratten kein Tier innerhalb der Nachbeobachtungszeit von 14 Tagen.

Aufgrund seiner pharmakologischen und pharmakokinetischen Eigenschaften ist das (S)-Enantiomere (AG-EE 623 ZW) und sind dessen physiologisch verträgliche Salze zur Behandlung des Diabetes mellitus geeignet. Hierzu lassen sich AG-EE 623 ZW oder dessen physiologisch verträgliche Salze, gegebenenfalls in Kombination mit anderen Wirksubstanzen, in die üblichen galenischen Zubereitungsformen wie Tabletten, Dragees, Kapseln, Pulver, Zäpfchen, Suspensionen oder Injektionslösungen einarbeiten. Die Einzeldosis am Erwachsenen beträgt hierbei 0.1 bis 20 mg, vorzugsweise 0.25 bis 5 mg, insbesondere jedoch 0.25, 0.5, 1.0, 1.5, 2.0, 2.5, 3.0 oder 5.0 mg, ein-, zwei- oder dreimal täglich.

Die nachfolgenden Beispiele sollen die Erfindung näher erläutern:

Beispiel A

<u>(S)-1-(2-Piperidino-phenyl)-3-methyl-1-butylamin</u>

Eine gerührte Lösung von 122 g (0,495 Mol) racemischem 1-(2-Piperidino-phenyl)-3-methyl-1-butylamin in 1000 ml Aceton wird mit 93,7 g (0,495 Mol) N-Acetyl-L-glutaminsäure versetzt. Man erhitzt auf dem Dampfbad unter Rückfluß und gibt portionsweise Methanol (insgesamt ca. 80 ml) zu, bis eine klare Lösung erreicht ist. Nach Abkühlen und Stehenlassen bei Raumtemperatur über Nacht saugt man die erhaltenen Kristalle ab, wäscht diese zweimal mit je 200 ml -15°C kaltem Aceton und trocknet diese. Das erhaltene Produkt [98,9 g; Schmelzpunkt: 163-166°C; $[\alpha]_D^{20}$ = + 0,286° (c = 1 in Methanol)] kristallisiert man aus 1000 ml Aceton unter Zusatz von 200 ml Methanol um, wobei man das (S)-1-(2-Piperidino-phenyl)-3-methyl-1-butylamin als N-Acetyl-L-glutaminsäure-Additionssalz erhält.
Ausbeute: 65,1 g (60,4 % der Theorie),
Schmelzpunkt: 168-171°C

| | | | | | | |
|---|---|---|---|---|---|---|
| Ber. | C | 63,42 | H | 8,56 | N | 9,65 |
| Gef. | | 63,64 | | 8,86 | | 9,60 |

$[\alpha]_D^{20}$ = + 0,357° (c = 1 in Methanol)

Das freie Amin erhält man als Öl durch Freisetzen beispielsweise mit einer Natriumhydroxid- oder Ammoniak-Lösung, Extraktion beispielsweise mit Toluol, Äther, Äthylacetat oder Methylenchlorid sowie Trocknen, Filtrieren und Eindampfen des Extraktes im Vakuum.

<u>Die (S)-Konfiguration</u> des Amins wurde wie folgt bewiesen: Umsetzung des Amins mit (S')-1-Phenäthylisocyanat in Äther zum entsprechenden Harnstoff-Derivat [Schmelzpunkt: 183-184°C; $[\alpha]_D^{20}$ = - 2,25° (c = 1 in Methanol)], Züchtung von Kristallen aus Äthanol/Wasser (8/1) und anschließende Röntgenstruktur-Analyse, die (S,S')-Konfiguration für das Harnstoff-Derivat und somit (S)-Konfiguration für das eingesetzte Amin ergab.

<u>Die Enantiomeren-Reinheit</u> wurde wie folgt bestimmt:

1. Acetylierung einer Probe des Amins mit 1,3 Äquivalenten Acetanhydrid in Eisessig bei 20°C über Nacht.
2. Untersuchung des N-Acetyl-Derivates (Schmelzpunkt: 128-132°C) mittels HPLC auf einer Chiral-Phasen-HPLC-Säule der Firma Baker, bei der (S)-N-(3,5-Dinitrobenzoyl)-2-phenyl-glycin kovalent an Aminopropyl-Kieselgel gebunden ist (Korngröße: 5 µm, kugelförmig, 60 A Porenweite; Säulenlänge: 250 mm bei 4,6 mm Innendurchmesser; Fließmittel: n-Hexan/Isopropanol (100/5); Fließgeschwindigkeit: 2 ml/Minute; Temperatur: 20°C; UV-Detektion bei 254 nm.

Gefunden: Peak 1(R): Peak 2(S) = 0,75 %: 99,25 %, ee (enantiomeric excess) = 98,5 % (S).

Mittels ätherischer Chlorwasserstoff-Lösung kann das (S)-Amin in sein Dihydrochlorid-hydrat übergeführt werden. Schmelzpunkt: 135-145°C (Zers.)

| | | | | | | | | |
|---|---|---|---|---|---|---|---|---|
| Ber. (x $H_2O$) | C | 56,99 | H | 8,97 | N | 8,31 | Cl | 21,02 |
| Gef. | | 56,85 | | 8,93 | | 8,38 | | 21,25 |

$[\alpha]_D^{20}$ = + 26,1° (c = 1 in Methanol)

Beispiel B

N-Acetyl-N-[1-(2-piperidino-phenyl)-3-methyl-1-buten-1-yl]-amin

Zu einer Lösung von 20 g (81,8 mMol) frisch hergestelltem Isobutyl-(2-piperidino-phenyl)-ketimin in 200 ml Acetonitril gibt man bei Raumtemperatur 4,7 ml (81,8 mMol) Eisessig, 25,7 g (98,2 mMol) Triphenylphosphin, 34,2 ml (245 mMol) Triäthylamin und 7,9 ml (81,8 mMol) Tetrachlorkohlenstoff und rührt 18 Stunden bei Raumtemperatur. Anschließend dampft man im Vakuum ein und verteilt zwischen Äthylacetat und Wasser. Den organischen Extrakt trocknet und filtriert man und dampft ihn im Vakuum ein. Den Eindampfrückstand reinigt man durch Säulenchromatographie an Kieselgel (Toluol/Äthylacetat = 10/1), wobei zuerst die (E)-Form und dann die (Z)-Form eluiert wird.
(E)-Form:
Ausbeute: 6,1 g (26 % der Theorie),
Schmelzpunkt: 135-137°C (Äthylacetat/Petroläther)

| | | | | | | |
|---|---|---|---|---|---|---|
| Ber. | C | 75,48 | H | 9,15 | N | 9,78 |
| Gef. | | 75,47 | | 9,35 | | 9,70 |

(Z)-Form:
Ausbeute: 3,1 g (13 % der Theorie),
Schmelzpunkt: 140-143°C (Äthylacetat)

| | | | | | | |
|---|---|---|---|---|---|---|
| Ber. | C | 75,48 | H | 9,15 | N | 9,78 |
| Gef. | | 75,56 | | 9,30 | | 9,79 |

Beispiel C

N-Acetyl-N-[1-(2-piperidino-phenyl)-3-methyl-1-buten-1-yl]-amin

Zu einer gerührten Lösung von 44 g (0,18 Mol) frisch hergestelltem Isobutyl-(2-piperidino-phenyl)-ketimin in 440 ml Toluol tropft man bei einer Innentemperatur von 0°C 17 ml (0,18 Mol) Acetanhydrid. Man rührt noch 3 Stunden bei 0°C und 15 Stunden bei Raumtemperatur, dampft dann im Vakuum ein, löst den Eindampfrückstand in Äthylacetat und schüttelt mehrmals mit wässriger Natriumhydrogencarbonat-Lösung aus. Man trocknet die organische Phase, filtriert sie und dampft sie im Vakuum ein. Den Eindampfrückstand reinigt man durch Säulenchromatographie an Kieselgel (Toluol/Äthylacetat = 5/1), wobei zuerst die (E)-Form und dann die (Z)-Form eluiert wird. (E)-Form:
Ausbeute: 3,0 g (5,8 % der Theorie),
(Z)-Form:
Ausbeute: 17,8 g (34,5 % der Theorie),
Schmelzpunkt: 139-141°C (Äthylacetat)

| | | | | | | |
|---|---|---|---|---|---|---|
| Ber. | C | 75,48 | H | 9,15 | N | 9,78 |
| Gef. | | 75,68 | | 8,99 | | 9,86 |

Beispiel D

N-Acetyl-N-[(S)-1-(2-piperidino-phenyl)-3-methyl-1-butyl]-amin

0,57 g (1,99 mMol) (Z)-N-Acetyl-N-[1-(2-piperidino-phenyl)-3-methyl-1-buten-1-yl]-amin vom Schmelzpunkt 139-141°C werden in 10 ml entgastem Lösungsmittelgemisch (Methanol/Methylenchlorid = 5/1) unter Argonatmosphäre gelöst und zu einer Lösung von 16,8 mg (1 Mol %) des NOYORI-Katalysators Ru(O-acetyl)$_2$[(S)-BINAP] (hergestellt aus [Ru(COD)Cl$_2$]$_n$ mit (S)-BINAP [= (S)-2,2'-Bis(diphenylphosphino)-1,1'-binaphthyl], Triäthylamin und Natriumacetat), und 3,4 mg (0,5 Mol %) Titantetraisopropylat in 10 ml entgastem Lösungsmittelgemisch (Methanol/Methylenchlorid = 5/1) gegeben. Das Reaktionsgemisch wird in einen bei $10^{-2}$ mbar evakuierten Autoklaven eingezogen. Man spült mehrmals mit Wasserstoff von 4 bar und hydriert anschließend bei 30°C und 100 bar bis zum Ende der Wasserstoffaufnahme (170 Stunden). Anschließend dampft man die braunrote Lösung im Vakuum ein, kocht den Eindampfrückstand mit 30 ml n-Hexan unter Rückfluß und filtriert heiß vom Ungelösten ab. Beim Abkühlen des Filtrates erfolgt Kristallisation.
Ausbeute: 0,31 g (54 % der Theorie),
Schmelzpunkt: 127-131°C
Enantiomeren-Reinheit: ee = 82 % (S) [HPLC-Methode: siehe Beispiel A].
Aus dem beim Kochen mit 30 ml n-Hexan erhaltenen Ungelösten läßt sich durch weiteres Auskochen mit n-Hexan, Filtration und Kristallisation aus der Hexan-Lösung 14 % des racemischen N-Acetyl-amins vom Schmelzpunkt 154-156°C gewinnen.

Beispiel E

(S)-1-(2-Piperidino-phenyl)-3-methyl-1-butylamin

Man erhitzt 1 g (3,47 mMol) N-Acetyl-N-[(S)-1-(2-piperidinophenyl)-3-methyl-1-butyl]-amin (Schmelzpunkt: 128-133°C; ee = 99,4 %] in 10 ml konzentrierter Salzsäure 5,5 Stunden unter Rückfluß, kühlt ab und gießt in ein Gemisch von konzentriertem Ammoniak und Eis. Man extrahiert zweimal mit Äthylacetat, wäscht die organische Phase mit Wasser, trocknet und filtriert sie und dampft sie im Vakuum ein.
Ausbeute: 0,84 g (98,8 % der Theorie) öliges Amin.

Durch Rückacetylierung mit 0,42 ml (1,3 Äquivalenten) Acetanhydrid in 8,4 ml Eisessig über Nacht bei Raumtemperatur, Eindampfen im Vakuum, Verteilung des Eindampfrückstandes zwischen Äthylacetat und gesättigter wässriger Natriumbicarbonat-Lösung sowie Trocknen, Filtrieren und Eindampfen des organischen Extraktes im Vakuum erhält man 0,83 g (84,7 % der Theorie) N-Acetyl-N-[(S)-1-(2-piperidino-phenyl)-3-methyl-1-butyl]-amin (Schmelzpunkt: 130-132°C; ee = 99,4 %).

Beispiel F

2-Äthoxy-4-[N-(1-(2-piperidino-phenyl)-3-methyl-1-buten-1-yl)-aminocarbonylmethyl]-benzoesäureäthylester

Hergestellt aus Isobutyl-(2-piperidino-phenyl)-ketimin und 3-Äthoxy-4-äthoxycarbonyl-phenylessigsäure analog Beispiel B. Reinigung durch Säulenchromatographie an Kieselgel (Toluol/ Aceton = 10/1), wobei zuerst die (E)-Form und dann die (Z)-Form eluiert wird.

(E)-Form:
Ausbeute: 4 % der Theorie,
Schmelzpunkt: 101-103°C

| | | | | | | |
|---|---|---|---|---|---|---|
| Ber. | C | 72,77 | H | 8,00 | N | 5,85 |
| Gef. | | 72,74 | | 7,78 | | 5,86 |

(Z)-Form:
Ausbeute: 28,1 % der Theorie,
Schmelzpunkt: 124-127°C (Petroläther/Toluol = 5/1)

| | | | | | | |
|---|---|---|---|---|---|---|
| Ber. | C | 72,77 | H | 8,00 | N | 5,85 |
| Gef. | | 72,90 | | 7,86 | | 5,83 |

Beispiel G

N-[(S')-1-Phenäthyl]-N-[(S)-1-(2-piperidino-phenyl)-3-methyl-1-butyl]-amin

17 g (49 mMol) N-[(S')-1-Phenäthyl]-isobutyl-(2-piperidinophenyl)-ketimin vom Siedepunkt 150-155°C/0,3 Torr [hergestellt aus Isobutyl-(2-piperidino-phenyl)-keton und (S')-1-Phenäthyl-amin (Fa. Fluka, ee = 99,6 %) in Toluol + Triäthylamin durch Zutropfen einer Lösung von Titantetrachlorid in Toluol] werden in 170 ml wasserfreiem Äthanol gelöst. Man setzt 1,7 g Titantetraisopropylat und 8 g Raney-Nickel zu und hydriert bei 50°C und 200 bar Wasserstoff. Nach 20 Stunden setzt man weitere 8 g Raney-Nickel zu und hydriert unter den gleichen Bedingungen weitere 52 Stunden. Man filtriert über eine Celite-Schicht auf einer G3-Fritte vom Katalysator ab und dampft das Filtrat im Vakuum ein.
Ausbeute: 13,1 g (76,6 % der Theorie),
Siedepunkt: 152°C/0,2 Torr

| | | | | | | |
|---|---|---|---|---|---|---|
| Ber. | C | 82,23 | H | 9,78 | N | 7,99 |
| Gef. | | 82,00 | | 10,03 | | 7,74 |

$[\alpha]_D^{20}$ = - 55,3° (c = 1,1 in Methanol)
Die Diastereomeren-Reinheit bestimmt man mittels HPLC auf einer Lichrosorb RP18-HPLC-Säule der Firma E. Merck (Deutschland); Säulenlänge: 250 mm bei einem Innendurchmesser von 4 mm; Teilchengröße; 7 µm. Fließmittel: Methanol/Dioxan/ 0,1 %wässrige Natriumacetat-Lösung, mit Essigsäure auf pH = 4,05 eingestellt (135/60/5); Temperatur: 23°C; UV-Detektion bei 254 nm.

Gefunden: Peak 1(S,S'): Peak 2(R,S') = 98,4 %: 1,4 %, de (diastereomeric excess) = 97,0 % (S,S').

Beispiel H

(S)-1-(2-Piperidino-phenyl)-3-methyl-1-butylamin

12,5 g (36 mMol) N-[(S')-1-Phenäthyl]-N-[(S)-1-(2-piperidinophenyl)-3-methyl-1-butyl]-amin mit einem de = 97,0 % (S,S') werden in 125 ml Wasser und 3,6 ml konz. Salzsäure gelöst. Man gibt 1,3 g Palladium/Kohle (10 %) zu und hydriert bei 50°C und 5 bar Wasserstoff. Nach beendeter Wasserstoffaufnahme (10 Stunden) filtriert man vom Katalysator über eine Celite-Schicht ab. Das Filtrat wird mit konz. Ammoniak unter Zusatz von Eis alkalisch gestellt und mit Äthylacetat extrahiert. Den organischen Extrakt trocknet und filtriert man und dampft ihn im Vakuum ein.
Ausbeute: 6,4 g (72,1 % der Theorie),
Siedepunkt: 115-117°C/0,4 Torr
Enantiomeren-Reinheit: ee = 93,5 % (S) [HPLC-Methode (nach vorangegangener Acetylierung): siehe Beispiel A].

Beispiel I

N-[(R')-1-Phenäthyl]-N-[(S)-1-(2-piperidino-phenyl)-3-methyl-1-butyl]-amin

Zu einer im Bad von 60°C gerührten Lösung von 27,4 mMol (4 Äquivalenten) Isobutyl-magnesiumbromid in 22 ml wasserfreiem Tetrahydrofuran tropft man die Lösung von 2 g (6,84 mMol) N-[(R')-1-Phenäthyl]-(2-piperidino-benzaldimin) [hergestellt aus äquimolaren Mengen 2-Piperidino-benzaldehyd und (R')-1-Phenäthylamin durch Stehen bei Raumtemperatur über Nacht und anschließende Trocknung mit Natriumsulfat in Äther-Lösung] in 20 ml wasserfreiem Tetrahydrofuran. Nach 18 Stunden erhöht man die Badtemperatur auf 80°C und gibt weitere 2 Äquivalente von Isobutyl-magnesiumbromid in 11 ml Tetrahydrofuran zu. Nach jeweils 12 Stunden Rühren bei 80°C gibt man noch einmal je 2 Äquivalente Isobutyl-magnesiumbromid-Lösung zu. Nach ca. 90 Stunden bei 80°C kühlt man ab, versetzt mit überschüssiger konz. Salzsäure und dampft im Wasserstrahlvakuum zur Trockne ein. Den Eindampfrückstand löst man in Wasser und alkalisiert mit konz. Ammoniak. Man extrahiert mit Äther, trocknet den organischen Extrakt über Natriumsulfat, filtriert ihn und dampft im Vakuum ein. Den Eindampfrückstand reinigt man durch Säulenchromatographie an Kieselgel (Toluol/Aceton = 95/5).
Ausbeute: 0,20 g (8,3 % der Theorie),
Schmelzpunkt: < 20°C
Die Diastereomeren-Reinheit wird wie im Beispiel G mittels HPLC bestimmt.

Gefunden: Peak 1(R,R'): Peak 2(S,R') = 4,4 %:95,6 %, de (diastereomeric excess) = 91,2 % (S,R').

Bei einem analogen Ansatz mit 2,0 g der Schiff'-Base und insgesamt 6 Äquivalenten Isobutyl-magnesiumbromid in Toluol/ Tetrahydrofuran (4/1) sowie unter Zusatz von 5 % Titan(IV)-tetraisopropylat und 60 Stunden Erhitzen bei 100°C in einer Glasbombe wurde eine Ausbeute von 5 % mit einem de = 97,6 % (S,R') erzielt.

Beispiel K

(S)-1-(2-Piperidino-phenyl)-3-methyl-1-butylamin

Man hydriert die Lösung von 0,15 g (0,428 mMol) N-[(R')-1-Phenäthyl]-N-[(S)-1-(2-piperidino-phenyl)-3-methyl-1-butyl]-amin (de = 91,2 %), 0,47 ml (0,47 mMol) 1N-Salzsäure und 1,5 ml Wasser in Gegenwart von 20 mg Palladium/Kohle (10%ig) 5 Stunden bei 50°C und 3,4 bar Wasserstoff. Man filtriert über Kieselgur, stellt mit konz. Ammoniak alkalisch und extrahiert mit Äthylacetat. Man trocknet den Extrakt, filtriert ihn und dampft im Vakuum ein.
Ausbeute: 0,066 g (62,8 % der Theorie),
Schmelzpunkt: < 20°C
Enantiomeren-Reinheit: ee = 87,6 % (S) [HPLC-Methode (nach vorangegangener Acetylierung): siehe Beispiel A].

Beispiel 1

(S)-2-Äthoxy-4-[N-(1-(2-piperidino-phenyl)-3-methyl-1-butyl)aminocarbonylmethyl]-benzoesäure-äthylester

Zu einer Lösung von 0,47 g (1,91 mMol) (S)-3-Methyl-1-(2-piperidino-phenyl)-1-butylamin (ee = 98,5 %) in 5 ml wasserfreiem Acetonitril gibt man nacheinander 0,48 g (1,91 mMol) 3-Äthoxy-4-äthoxycarbonyl-phenylessigsäure, 0,60 g (2,29 mMol) Triphenylphosphin, 0,80 ml (5,73 mMol) Triäthylamin und 0,18 ml (1,91 mMol) Tetrachlorkohlenstoff und rührt 20 Stunden bei Raumtemperatur. Anschließend dampft man im Vakuum ein und verteilt zwischen Äthylacetat und Wasser. Den organischen Extrakt trocknet und filtriert man und dampft ihn im Vakuum ein. Den Eindampfrückstand reinigt man durch Säulenchromatographie an Kieselgel (Toluol/Äthylacetat = 10/1).
Ausbeute: 0,71 g (77,3 % der Theorie),
Schmelzpunkt: 110-112°C

| | | | | | | | |
|---|---|---|---|---|---|---|---|
| Ber. | C | 72,47 | H | 8,39 | N | 5,83 |
| Gef. | | 72,29 | | 8,42 | | 5,80 |

Die Enantiomeren-Reinheit bestimmt man mittels HPLC auf einer Chiral-Phasen-HPLC-Säule der Firma Baker, bei der (S)-N-3,5-Dinitrobenzoyl-leucin kovalent an Aminopropyl-Kieselgel gebunden ist (Korngröße: 5 µm, kugelförmig, 60 A Porenweite; Säulenlänge: 250 mm bei einem Innendurchmesser von 4,6 mm; Fließmittel: n-Hexan/Tetrahydrofuran/Methylenchlorid/Äthanol (90/10/1/1); Fließgeschwindigkeit: 2 ml/Minute; Temperatur: 20°C; UV-Detektion bei 242 nm).

Gefunden: Peak 1(R): Peak 2(S) = 0,75 %: 99,25 %, ee = 98,5 % (S).

Beispiel 2

(S)-2-Äthoxy-4-[N-(1-(2-piperidino-phenyl)-3-methyl-1-butyl)-aminocarbonylmethyl]-benzoesäure-äthylester

Zu einer Lösung von 2,71 g (11 mMol) wasserfreiem (S)-3-Methyl-1-(2-piperidino-phenyl)-1-butylamin (ee = 98,5 %) in 30 ml absolutem Toluol gibt man bei Raumtemperatur 2,77 g (11 mMol) 3-Äthoxy-4-äthoxycarbonyl-phenylessigsäure und rührt, bis Lösung erreicht ist. Dann gibt man 2,38 g (11,55 mMol) N,N'-Dicyclohexyl-carbodiimid zu und rührt bei Raumtemperatur. Nach 24 Stunden setzt man weitere 0,54 g (2,14 mMol) 3-Äthoxy-4-äthoxycarbonyl-phenylessigsäure und 0,48 g (2,33 mMol) N,N'-Dicyclohexyl-carbodiimid zu und rührt über Nacht. Anschließend kühlt man auf eine Innentemperatur von +5°C ab und nutscht vom ausgefallenen Niederschlag ab, den man einmal mit 5 ml Toluol wäscht. Die vereinigten Toluol-Filtrate engt man im Vakuum auf ein Volumen von ca. 10 ml ein. Die so erhaltene Lösung erhitzt man auf dem Dampfbad und versetzt sie portionsweise mit Petroläther (insgesamt 55 ml) bis zur bleibenden Trübung. Man kühlt in Eis ab, wobei Kristallisation erfolgt. Man nutscht ab und trocknet bei 75°C/ 4 Torr. Das erhaltene Produkt (4,57 g; Schmelzpunkt 111-112°C; ee = 98,9 %) suspendiert man in 50 ml Petroläther. Man erhitzt auf dem Dampfbad und gibt portionsweise soviel Toluol (insgesamt 8 ml) zu, bis Lösung erzielt ist. Dann kühlt man in

Eis ab und nutscht vom Kristallisat ab, das man bei 75°C/4 Torr trocknet.
Ausbeute: 3,93 g (74,3 % der Theorie),
Schmelzpunkt: 117-118°C

| | | | | | | |
|---|---|---|---|---|---|---|
| Ber. | C | 72,47 | H | 8,39 | N | 5,83 |
| Gef. | | 72,44 | | 8,43 | | 5,93 |

$[\alpha]_D^{20}$ = + 9,4° (c = 1,01 in Methanol)
Enantiomeren-Reinheit: ee = 99,9 % [HPLC-Methode: siehe Beispiel 1]

Beispiel 3

(S)-2-Äthoxy-4-[N-(1-(2-piperidino-phenyl)-3-methyl-1-butyl)-aminocarbonylmethyl]-benzoesäure

Man rührt die Lösung von 3,79 g (7,88 mMol) (S)-2-Äthoxy-4-[N-(1-(2-piperidino-phenyl)-3-methyl-1-butyl)-aminocarbonylmethyl]-benzoesäure-äthylester (ee = 99,9 %) in 37 ml Äthanol im Bad von 60°C und setzt 10 ml (10 mMol) 1N-Natronlauge zu. Nach 4 Stunden Rühren bei 60°C gibt man in der Wärme 10 ml (10 mMol) 1N-Salzsäure zu und läßt auf Raumtemperatur abkühlen. Nach Animpfen und Stehenlassen über Nacht kühlt man noch für eine Stunde unter Rühren in Eis ab. Man nutscht vom Kristallisat ab und wäscht es zweimal mit je 5 ml Wasser. Anschließend trocknet man bei 75°C bis zuletzt 100°C/4 Torr im Vakuum-Trockenschrank über Phosphorpentoxid.
Ausbeute: 3,13 g (87,7 % der Theorie),
Schmelzpunkt: 130-131°C (hochschmelzende Form)

| | | | | | | |
|---|---|---|---|---|---|---|
| Ber. | C | 71,64 | H | 8,02 | N | 6,19 |
| Gef. | | 71,48 | | 7,87 | | 6,39 |

$[\alpha]_D^{20}$ = + 7,45° (c = 1,06 in Methanol)
Die Enantiomeren-Reinheit bestimmt man mittels HPLC auf einer Chiral-Phasen-HPLC-Säule der Firma ChromTech (Schweden) mit einer AGP(α1-acid glycoprotein)-Phase; Innendurchmesser: 4,0 mm; Länge: 100 mm; Teilchendurchmesser: 5 µm. Temperatur: 20°C; Fließmittel: 0,1 % wässrige $KH_2PO_4$-Lösung (=A) + 20 % Acetonitril (=B), Gradientensteigerung binnen 4 Minuten auf 40 % (B); Fließgeschwindigkeit: 1 ml/Minute; UV-Detektion bei 240 nm. Retentionszeit (S)-Enantiomer: 2,7 Minuten; Retentionszeit (R)-Enantiomer: 4,1 Minuten.

Gefunden: (S):(R) = 99,85 %: 0,15 %, ee = 99,7 % (S).

Bei Rekristallisation einer Probe aus Äthanol/Wasser (2/1) verändert sich der Schmelzpunkt nicht.
Bei Erhitzen einer Probe in Petroläther/Toluol (5/3), Abfiltrieren des ungelösten Anteils (Schmelzpunkt: 130-131°C) und raschem Abkühlen des Filtrats erhält man die niedrigschmelzende Form der Titelverbindung vom Schmelzpunkt 99-101°C.

| | | | | | | |
|---|---|---|---|---|---|---|
| Ber. | C | 71,64 | H | 8,02 | N | 6,19 |
| Gef. | | 71,66 | | 7,97 | | 6,44 |

Die niedrigschmelzende Form und die hochschmelzende Form unterscheiden sich in ihren Infrarot-KBr-Spektren, jedoch nicht in ihren Infrarot-Lösungs-Spektren (Methylenchlorid).
Erhitzt man eine Probe der niedrigschmelzenden Form über ihren Schmelzpunkt hinaus, so beobachtet man einen zweiten Schmelzpunkt bei 127-130°C.
Kristallisiert man eine Probe der niedrigschmelzenden Form aus Äthanol/Wasser (2/1) um, so erhält man die hochschmelzende Form.
Die hochschmelzende Form und die niedrigschmelzende Form wurden mittels "Differential Scanning Calorimetry (DSC)" [Apparat Mettler, TA-300-System; Meßzelle: DSC 20; Fa. Mettler, CH-8306 Greifensee, Schweiz] mit folgendem Ergebnis untersucht:

| Verbindung aus Beispiel 3 | Aufheizrate 10°K/Min. | Aufheizrate 3°K/Min. |
|---|---|---|
| Hochschmelzende Form | Einheitlicher Schmelzpeak mit Schmelztemperatur von 133°C; Schmelzenthalpie: 100 J/g | Einheitlicher Schmelzpeak mit Schmelztemperatur von 132°C; Schmelzenthalpie: 99,1 J/g |
| Niedrigschmelzende Form | 1. Peak bei 57°C (sehr schwach)<br>2. Peak bei 78°C (schwach)<br>3. endothermer Peak bei 107°C; Schmelzenthalpie: 55 J/g<br>4. endothermer Peak bei 132°C Schmelzenthalpie: 25 J/g | 1. Peak bei 54°C (sehr schwach; endotherm)<br>2. endothermer Peak bei 104°C, Schmelztemperatur 102°C, Schmelzenthalpie 52 J/g<br>3. exothermer Verlauf der Basislinie durch Auskristallisieren der bei 104°C geschmolzenen Substanz<br>4. endothermer Peak bei 131°C, Schmelztemperatur: 130°C Schmelztemperatur 52 J/g |

Beispiel 4

(S)-2-Äthoxy-4-[N-(1-(2-piperidino-phenyl)-3-methyl-1-butyl)-aminocarbonylmethyl]-benzoesäure-äthylester

0,79 g (1,65 mMol) (Z)-2-Äthoxy-4-[N-(1-(2-piperidino-phenyl)-3-methyl-1-buten-1-yl)-aminocarbonylmethyl]-benzoesäureäthylester vom Schmelzpunkt 124-127°C werden in 10 ml entgastem Lösungsmittelgemisch (Methanol/Methylenchlorid = 5/1) unter Argonatmosphäre gelöst und zu einer Lösung von 17 mg des NOYORI-Katalysators Ru (0-acetyl)$_2$[(S)-BINAP] (hergestellt aus $[Ru(COD)Cl_2]_n$ mit (S)-BINAP [= (S)-2,2'-Bis-(diphenylphosphino)-1,1'-binaphthyl], Triäthylamin und Natriumacetat) und 3 mg Titantetraisopropylat in 10 ml entgastem Lösungsmittelgemisch (Methanol/Methylenchlorid = 5/1) gegeben. Das Reaktionsgemisch wird in einen bei $10^{-2}$ mbar evakuierten Autoklaven eingezogen. Man spült fünfmal mit Wasserstoff von 5 bar und hydriert anschließend bei 30°C und 100 bar bis zum Ende der Wasserstoffaufnahme (154 Stunden). Man dampft die braunrote Lösung im Vakuum ein, löst den Eindampfrückstand in 80 ml Äther, filtriert von ungelösten braunen Flocken mittels Aktivkohle ab und dampft das so erhaltene klare hellgelbe Filtrat im Vakuum ein. Den Eindampfrückstand (0,60 g) kocht man in 60 ml n-Hexan unter Rückfluß und filtriert heiß vom Ungelösten ab. Das Filtrat läßt man über Nacht bei Raumtemperatur stehen. Von den dabei abgeschiedenen Kristallen filtriert man ab.
Ausbeute: 0,45 g (56,7 % der Theorie),
Schmelzpunkt: 131-133°C (nach Sintern ab 120°C)
Enantiomeren-Reinheit: ee = 39 % (S) [HPLC-Methode: siehe Beispiel 1].

Beispiel 5

(S)-2-Äthoxy-4-[N-(1-(2-piperidino-phenyl)-3-methyl-1-butyl)-aminocarbonylmethyl]-benzoesäure-äthylester

Zu einer Lösung von 0,68 g (1,15 mMol) (S)-2-Hydroxy-4-[N-(1-(2-piperidino-phenyl)-3-methyl-1-butyl)aminocarbonylmethyl]-benzoesäure-äthylester [Schmelzpunkt: 125-126°C; $[\alpha]_D^{20}$ = + 12,87° (c = 1,01 in Methanol)] in 5 ml wasserfreiem Dimethylformamid gibt man 0,05 g (1,15 mMol) Natriumhydrid/55%ig in Öl und rührt 0,5 Stunden bei Raumtemperatur. Dann tropft man die Lösung von 0,12 ml (1,15 mMol) Äthyljodid in 2,5 ml wasserfreiem Dimethylformamid zu und rührt 5 Stunden bei Raumtemperatur. Man dampft im Vakuum ein, verteilt zwischen verdünnter Natronlauge und Chloroform, trocknet den organischen Extrakt, filtriert ihn und dampft ihn im Vakuum ein. Den Eindampfrückstand reinigt man durch Säulenchromatographie an Kieselgel (Toluol/Äthylacetat = 10/1).
Ausbeute: 0,48 g (67 % der Theorie),
Schmelzpunkt: 110-112°C

| | | | | | | |
|---|---|---|---|---|---|---|
| Ber. | C | 72,47 | H | 8,39 | N | 5,83 |
| Gef. | | 72,61 | | 8,54 | | 5,97 |

Enantiomeren-Reinheit: ee = 98,5 % (S) [HPLC-Methode: siehe Beispiel 1].

Beispiel 6

(S)-2-Äthoxy-4-[N-(1-(2-piperidino-phenyl)-3-methyl-1-butyl)-aminocarbonylmethyl]-benzoesäure-äthylester

Hergestellt aus (S)-2-Hydroxy-4-[N-(1-(2-piperidino-phenyl)-3-methyl-1-butyl)-aminocarbonylmethyl]-benzoesäure analog Beispiel 5 mittels 2 Äquivalenten Natriumhydrid und 2 Äquivalenten Äthyljodid.
Ausbeute: 42 % der Theorie,
Schmelzpunkt: 110-112°C

| | | | | | | |
|---|---|---|---|---|---|---|
| Ber. | C | 72,47 | H | 8,39 | N | 5,83 |
| Gef. | | 72,61 | | 8,54 | | 5,99 |

Enantiomeren-Reinheit: ee = 98,3 % (S) [HPLC-Methode: siehe Beispiel 1].

Beispiel 7

(S)(+)-2-Äthoxy-4-[N-(1-(2-piperidino-phenyl)-3-methyl-1-butyl)-aminocarbonylmethyl]-benzoesäure-äthylester und (R)(-)-2-Äthoxy-4-[N-(1-(2-piperidino-phenyl)-3-methyl-1-butyl)-aminocarbonylmethyl]-benzoesäure-äthylester

920 mg (±)-2-Äthoxy-4-[N-(1-(2-piperidino-phenyl)-3-methyl-1-butyl)-aminocarbonylmethyl]-benzoesäure-äthylester werden in Einzeldosen à 10 mg auf einer präparativen Chiral-Phasen-HPLC-Säule der Firma Baker aufgetrennt, bei der (S)-N-3,5-Dinitrobenzoyl-leucin kovalent an Aminopropyl-Kieselgel gebunden ist (Korngröße: 40 µm; Säulenlänge: 250 mm bei einem Innendurchmesser von 20 mm; Fließmittel: n-Hexan/Tetrahydrofuran/Äthanol/Methylenchlorid (180/20/3/2); Fließgeschwindigkeit: 21,25 ml/Minute; Temperatur: 27°C; UV-Detektion bei 285 nm), wobei zuerst das (R)(-)Enantiomer (Peak 1) und dann das (S)(+)Enantiomer (Peak 2) eluiert wird.
Aus dem entsprechend geschnittenen und gesammelten Fraktionen werden nach Eindampfen im Vakuum erhalten:
Fraktion "Peak 1" (R): 423 mg (roh),
Fraktion "Peak 2" (S): 325 mg (roh).

Zur Abtrennung von Verunreinigungen (u.a. des im Tetrahydrofuran enthaltenen Stabilisators 2,6-Di-tert.butyl-4-methylphenol) werden die beiden Fraktionen jeweils säulenchromatographisch an Kieselgel (Toluol/Aceton = 10/1) gereinigt.

(R)(-)-Enantiomer:
Ausbeute: 234,5 mg (51 % der Theorie),
Schmelzpunkt: 122-124°C (Petroläther + Aceton)

| | | | | | | |
|---|---|---|---|---|---|---|
| Ber. | C | 72,47 | H | 8,39 | N | 5,83 |
| Gef. | | 72,40 | | 8,18 | | 5,71 |

$[\alpha]_D^{20}$ = - 8,3° (c = 1 in Methanol)

(S)-Enantiomer:
Ausbeute: 131,2 mg (28,5 % der Theorie),
Schmelzpunkt: 122-124°C (Petroläther/Aceton = 8/1)

| | | | | | | |
|---|---|---|---|---|---|---|
| Ber. | C | 72,47 | H | 8,39 | N | 5,83 |
| Gef. | | 72,28 | | 8,44 | | 5,70 |

$[\alpha]_D^{20}$ = + 8,3° (c = 1 in Methanol)

Zur Trennung der Enantiomeren ist auch eine Chiralcel OD-Säule der Firma Daicel geeignet. Auf einer Säule von 250 mm Länge und 4,6 mm Innendurchmesser (Fließmittel: absolutes Äthanol/(n-Hexan + 0,2 % Diäthylamin) = 5/95; Temperatur: 40°C; UV-Detektion bei 245 nm) wird das (R)-Enantiomere nach 6,8 Minuten und das (S)-Enantiomere nach 8,5 Minuten eluiert.

Beispiel 8

(R)(-)-2-Äthoxy-4-[N-(1-(2-piperidino-phenyl)-3-methyl-1-butyl)-aminocarbonylmethyl]-benzoesäure x 0,4 $H_2O$

Hergestellt aus 150 mg (0,312 mMol) (R)(-)-2-Äthoxy-4-[N-(1-(2-piperidino-phenyl)-3-methyl-1-butyl)-aminocarbonylmethyl]-benzoesäure-äthylester [Schmelzpunkt: 122-124°C;
$[\alpha]_D^{20}$ = - 8,3° (c = 1 in Methanol)] durch Verseifung mit 1N-Natronlauge in Äthanol analog Beispiel 3.
Ausbeute: 95,8 mg (66,7 % der Theorie),
Schmelzpunkt: 103-105°C (Toluol/Petroläther)

| | | | | | | |
|---|---|---|---|---|---|---|
| Ber. (x 0,4 $H_2O$) | C | 70,51 | H | 8,01 | N | 6,09 |
| Gef. | | 70,88 | | 7,79 | | 5,81 |

Molpeak $M^+$: Ber.: 452
Gef.: 452

$[\alpha]_D^{20}$ = - 6,5° (c = 1 in Methanol)
Enantiomeren-Reinheit: ee = 99,7 % (R) [HPLC-Methode: siehe Beispiel 3].

Beispiel 9

(S)(+)-2-Äthoxy-4-[N-(1-(2-piperidino-phenyl)-3-methyl-1-butyl)-aminocarbonylmethyl]-benzoesäure x 0,4 $H_2O$

Hergestellt aus 89 mg (0,198 mMol) (S)(+)-2-Äthoxy-4-[N-(1-(2-piperidino-phenyl)-3-methyl-1-butyl)-aminocarbonylmethyl]-benzoesäure-äthylester [Schmelzpunkt: 122-124°C;
$[\alpha]_D^{20}$ = + 8,3° (c = 1 in Methanol)] durch Verseifung mit 1N-Natronlauge in Äthanol analog Beispiel 3.
Ausbeute: 44,5 mg (48,8 % der Theorie),
Schmelzpunkt: 102-103°C (Toluol/Petroläther)

| | | | | |
|---|---|---|---|---|
| Ber.: (x 0,4 $H_2O$) | C | 70,51 | H | 8,01 |
| Gef.: | | 70,80 | | 8,06 |

$[\alpha]_D^{20}$ = + 6,7° (c = 1 in Methanol)
Enantiomeren-Reinheit: ee = 99,6 % (S) [HPLC-Methode: siehe Beispiel 3].

Beispiel 10

(S)-2-Äthoxy-4-[N-(1-(2-piperidino-phenyl)-3-methyl-1-butyl)-aminocarbonylmethyl]-benzoesäure

Man hydriert 0,26 g (0,47 mMol) (S)-2-Äthoxy-4-[N-(1-(2-piperidino-phenyl)-3-methyl-1-butyl)-aminocarbonylmethyl]-benzoesäure-benzylester (Schmelzpunkt: 91-92°C;
$[\alpha]_D^{20}$ = + 9,5° ; c = 1,05 in Methanol) in 10 ml Äthanol an 0,12 g Palladium/Kohle (10%ig) bei 50°C und 5 bar Wasserstoff. Nach 5 Stunden filtriert man den Katalysator über Kieselgur ab und dampft im Vakuum ein. Den Eindampfrückstand kristallisiert man aus Äthanol/Wasser (2/1).
Ausbeute: 0,15 g (70 % der Theorie),
Schmelzpunkt: 130-131°C

| | | | | | | |
|---|---|---|---|---|---|---|
| Ber. | C | 71,64 | H | 8,02 | N | 6,19 |
| Gef. | | 71,76 | | 8,12 | | 6,05 |

Enantiomeren-Reinheit: ee = 99,6 % [HPLC-Methode: siehe Beispiel 3].

Beispiel 11

(S)-2-Äthoxy-4-[N-(1-(2-piperidino-phenyl)-3-methyl-1-butyl)-aminocarbonylmethyl]-benzoesäure

Man erhitzt 102 mg (0,20 mMol) (S)-2-Äthoxy-4-[N-(1-(2-piperidino-phenyl)-3-methyl-1-butyl)-aminocarbonylmethyl]-benzoesäure-tert.butylester (Schmelzpunkt: 122-123°C; $[\alpha]_D^{20}$ = + 8,7° ; c = 1 in Methanol) in 5 ml Benzol zusammen auf einigen Kristallen p-Toluolsulfonsäure-hydrat einen halben Tag auf Rückfluß. Dann ist laut Dünnschichtchromatogramm nach $R_F$-Wert und Massenspektrum das gewünschte Produkt entstanden.
Schmelzpunkt: 129-131°C

| | |
|---|---|
| Molpeak $M^+$: | Ber.: 452 |
| | Gef.: 452 |

Beispiel 12

(S)-2-Äthoxy-4-[N-(1-(2-piperidino-phenyl)-3-methyl-1-butyl)-aminocarbonylmethyl]-benzoesäure

Man rührt 200 mg (0,395 mMol) (S)-2-Äthoxy-4-[N-(1-(2-piperidino-phenyl)-3-methyl-1-butyl)-aminocarbonylmethyl]-benzoesäure-tert.butylester (Schmelzpunkt: 122-123°C; $[\alpha]_D^{20}$ = + 8,7° ; c = 1 in Methanol) in 2 ml Methylenchlorid zusammen mit 0,45 g (3,95 mMol) Trifluoressigsäure über Nacht bei Raumtemperatur. Man dampft im Vakuum ein und verteilt den Eindampfrückstand zwischen wässriger Natriumhydrogencarbonat-Lösung und Äthylacetat. Man trocknet den organischen Extrakt, filtriert ihn und dampft im Vakuum ein. Den Eindampfrückstand kristallisiert man aus Äthanol/Wasser (2/1).
Ausbeute: 115 mg (64,7 % der Theorie),
Schmelzpunkt: 126-128°C

| | | | | | | |
|---|---|---|---|---|---|---|
| Ber. | C | 71,64 | H | 8,02 | N | 6,19 |
| Gef. | | 71,39 | | 7,91 | | 6,06 |

$[\alpha]_D^{20}$ = + 6,97° (c = 0,975 in Methanol)
Enantiomeren-Reinheit: ee = 99,8 % [HPLC-Methode: siehe Beispiel 3].

Beispiel 13

| Tabletten mit 0,25 mg AG-EE 623 ZW | |
|---|---|
| Eine Tablette enthält: | |
| 0,250 mg | Wirksubstanz |
| 0,125 mg | N-Methylglucamin |
| 0,038 mg | Polyvinylpyrrolidon |
| 0,075 mg | Polyoxyäthylenpolyoxypropylenpolymer |
| 0,150 mg | mikrokristalline Cellulose |

Herstellung:

Wirk- und Hilfsstoffe werden in Wasser von 90°C gelöst bzw. die mikrokristalline Cellulose suspendiert und die Dispersion im Vakuum eingedampft. Die trockene Masse wird auf 1 mm Maschenweite gesiebt.

Dem Wirkstoffgranulat werden pro Tablette folgende Bestandteile zugemischt:

| | |
|---|---|
| 24,682 mg | Natrium-carboxymethylstärke |
| 24,000 mg | mikrokristalline Cellulose |
| 0,500 mg | Magnesiumstearat |
| 50,000 mg | |

Aus dieser Mischung werden runde, biplane Tabletten von 50 mg und 5 mm Durchmesser gepreßt.

Beispiel 14

| Tabletten mit 0,5 mg AG-EE 623 ZW | |
|---|---|
| Eine Tablette enthält: | |
| 0,500 mg | Wirksubstanz |
| 0,250 mg | N-Methylglucamin |
| 0,075 mg | Polyvinylpyrrolidon |
| 0,150 mg | Polyoxyäthylenpolyoxypropylenpolymer |
| 0,300 mg | mikrokristalline Cellulose |

Herstellung:

Wirk- und Hilfsstoffe werden in Wasser von 90°C gelöst bzw. die mikrokristalline Cellulose suspendiert und die Dispersion im Vakuum eingedampft. Die trockene Masse wird auf 1 mm Maschenweite gesiebt.

Dem Wirkstoffgranulat werden pro Tablette folgende Bestandteile zugemischt:

| | |
|---|---|
| 24,225 mg | Natrium-carboxymethylstärke |
| 24,000 mg | mikrokristalline Cellulose |
| 0,500 mg | Magnesiumstearat |
| 50,000 mg | |

Aus dieser Mischung werden runde, biplane Tabletten von 50 mg und 5 mm Durchmesser gepreßt.

Beispiel 15

| Tabletten mit 1,0 mg AG-EE 623 ZW | |
|---|---|
| Eine Tablette enthält: | |
| 1,00 mg | Wirksubstanz |
| 0,50 mg | N-Methylglucamin |
| 0,15 mg | Polyvinylpyrrolidon |
| 0,03 mg | Polyoxyäthylenpolyoxypropylenpolymer |
| 0,60 mg | mikrokristalline Cellulose |

Herstellung:

Wirk- und Hilfsstoffe werden in Wasser von 90°C gelöst bzw. die mikrokristalline Cellulose suspendiert und die Dispersion im Vakuum eingedampft. Die trockene Masse wird auf 1 mm Maschenweite gesiebt.

Dem Wirkstoffgranulat werden pro Tablette folgende Bestandteile zugemischt:

| | |
|---|---|
| 23,22 mg | Natrium-carboxymethylstärke |
| 24,00 mg | mikrokristalline Cellulose |
| 0,50 mg | Magnesiumstearat |
| 50,00 mg | |

Aus dieser Mischung werden runde, biplane Tabletten von 50 mg und 5 mm Durchmesser gepreßt.

Beispiel 16

| Tabletten mit 1,5 mg AG-EE 623 ZW | |
|---|---|
| Eine Tablette enthält: | |
| 1,500 mg | Wirksubstanz |
| 0,750 mg | N-Methylglucamin |
| 0,225 mg | Polyvinylpyrrolidon |
| 0,045 mg | Polyoxyäthylenpolyoxypropylenpolymer |
| 0,900 mg | mikrokristalline Cellulose |

Herstellung:

Wirk- und Hilfsstoffe werden in Wasser von 90°C gelöst bzw. die mikrokristalline Cellulose suspendiert und die Dispersion im Vakuum eingedampft. Die trockene Masse wird auf 1 mm Maschenweite gesiebt.

Dem Wirkstoffgranulat werden pro Tablette folgende Bestandteile zugemischt:

| | |
|---|---|
| 23,080 mg | Natrium-carboxymethylstärke |
| 23,000 mg | mikrokristalline Cellulose |
| 0,500 mg | Magnesiumstearat |
| 50,000 mg | |

Aus dieser Mischung werden runde, biplane Tabletten von 50 mg und 5 mm Durchmesser gepreßt.

Beispiel 17

| Tabletten mit 2,0 mg AG-EE 623 ZW | |
|---|---|
| Eine Tablette enthält: | |
| 2,00 mg | Wirksubstanz |
| 1,00 mg | L-Lysin |
| 1,00 mg | Polyvinylpyrrolidon |
| 1,00 mg | Polyoxyäthylenpolyoxypropylenpolymer |
| 4,00 mg | mikrokristalline Cellulose |

Herstellung:

Die Bestandteile werden in Wasser von 90°C gelöst bzw. die mikrokristalline Cellulose suspendiert und die Dispersion in einem Sprühtrockner verarbeitet. Anschließend werden pro Tablette folgende Bestandteile zugefügt:

| | |
|---|---|
| 20,35 mg | mikrokristalline Cellulose |
| 20,00 mg | Natrium-carboxymethylstärke |
| 0,65 mg | Magnesiumstearat |
| 50,00 mg | |

Aus dieser Mischung werden runde, bikonvexe Tabletten von 50 mg und 5 mm Durchmesser gepreßt und mit Hydroxypropylmethylcellulose geschmacksabdeckend überzogen.

Beispiel 18

| Tabletten mit 2,5 mg AG-EE 623 ZW | |
|---|---|
| Eine Tablette enthält: | |
| 2,50 mg | Wirksubstanz |
| 1,25 mg | L-Lysin |
| 1,25 mg | Polyvinylpyrrolidon |
| 1,25 mg | Polyoxyäthylenpolyoxypropylenpolymer |
| 4,10 mg | mikrokristalline Cellulose |

Herstellung:

Die Bestandteile werden in Wasser von 90°C gelöst bzw. die mikrokristalline Cellulose suspendiert und die Dispersion in einem Sprühtrockner verarbeitet. Anschließend werden pro Tablette folgende Bestandteile zugefügt:

| | |
|---|---|
| 19,50 mg | mikrokristalline Cellulose |
| 19,50 mg | Natrium-carboxymethylstärke |
| 0,65 mg | Magnesiumstearat |
| 50,00 mg | |

Aus dieser Mischung werden runde, bikonvexe Tabletten von 50 mg und 5 mm Durchmesser gepreßt und mit Hydroxypropylmethylcellulose geschmacksabdeckend überzogen.

Beispiel 19

| Tabletten mit 3,0 mg AG-EE 623 ZW | |
|---|---|
| Eine Tablette enthält: | |
| 3,0 mg | Wirksubstanz |
| 1,5 mg | L-Lysin |
| 1,5 mg | Polyvinylpyrrolidon |
| 1,5 mg | Polyoxyäthylenpolyoxypropylenpolymer |

Herstellung:

Die Bestandteile werden in Wasser von 90°C gelöst und die Lösung in einem Sprühtrockner verarbeitet. Anschließend werden pro Tablette folgende Bestandteile zugefügt:

| | |
|---|---|
| 21,5 mg | mikrokristalline Cellulose |
| 21,0 mg | Natrium-carboxymethylstärke |
| 50,0 mg | |

Aus dieser Mischung werden runde, bikonvexe Tabletten von 50 mg und 5 mm Durchmesser gepreßt und mit Hydroxypropylmethylcellulose geschmacksabdeckend überzogen.

Beispiel 20

| Tabletten mit 5 mg AG-EE 623 ZW | |
|---|---|
| Eine Tablette enthält: | |
| 5,0 mg | Wirksubstanz |
| 2,5 mg | L-Lysin |
| 2,5 mg | Polyvinylpyrrolidon |
| 2,5 mg | Polyoxyäthylenpolyoxypropylenpolymer |

Herstellung:

Die Bestandteile werden in Wasser von 90°C gelöst und die Lösung in einem Sprühtrockner verarbeitet. Anschließend werden pro Tablette folgende Bestandteile zugefügt:

| | |
|---|---|
| 19,0 mg | mikrokristalline Cellulose |
| 18,5 mg | Natrium-carboxymethylstärke |
| 50,0 mg | |

Aus dieser Mischung werden runde, bikonvexe Tabletten von 50 mg und 5 mm Durchmesser gepreßt und mit Hydroxypropylmethylcellulose geschmacksabdeckend überzogen.

## Patentansprüche

1. Verwendung von (S) (+)-2-Äthoxy-4-[N-[1-(2-piperidino-phenyl)-3-methyl-1-butyl]aminocarbonylmethyl]-benzoesäure als Wirkstoff oder eines physiologisch verträglichen Salzes hiervon zur Herstellung eines Langzeitantidiabeticums,
dadurch gekennzeichnet, daß im Vergleich zu der doppelten Einzeldosis bei einer Racemat-Applikation unnötig hohe und langandauernde Substanzbelastungen vermieden werden, wodurch wesentlich niedrigere Wirkstoff-Plasmaspiegel auftreten, die über den normalen Vorteil der Dosis-Halbierung bei der Enantiomeren-Applikation hinausgehen.

2. Verwendung von (S)(+)-2-Äthoxy-4-[N-[1-(2-piperidino-phenyl)-3-methyl-1-butyl]aminocarbonylmethyl]-benzoesäure gemäß Anspruch 1, dadurch gekennzeichnet, daß der Wirkstoff mit einer optischen Reinheit von mindestens ee = 95 oder ein physiologisch verträgliches Salz hiervon eingesetzt wird.

3. Verwendung von (S)(+)-2-Äthoxy-4-[N-[1-(2-piperidino-phenyl)-3-methyl-1-butyl]aminocarbonylmethyl]-benzoesäure gemäß Anspruch 1, dadurch gekennzeichnet, daß der Wirkstoff mit einer optischen Reinheit von mindestens ee = 98 oder ein physiologisch verträgliches Salz hiervon eingesetzt wird.

4. Verwendung von (S)(+)-2-Äthoxy-4-[N-[1-(2-piperidino-phenyl)-3-methyl-1-butyl]aminocarbonylmethyl]-benzoesäure als Wirkstoff oder ein physiologisch verträgliches Salz hiervon zur Herstellung eines Arzneimittels gemäß Anspruch 1, 2 oder 3, dadurch gekennzeichnet, daß die Einzeldosis in einem Bereich von 0.25 bis 5.0 mg liegt.

5. Verwendung nach Anspruch 4, dadurch gekennzeichnet, daß die Einzeldosis 0.5 mg beträgt.

6. Verwendung nach Anspruch 4, dadurch gekennzeichnet, daß die Einzeldosis 1.0 mg beträgt.

7. Verwendung nach Anspruch 4, dadurch gekennzeichnet, daß die Einzeldosis 2.0 mg beträgt.

## Claims

1. Use of (S)(+)-2-ethoxy-4-[N-[1-(2-piperidinophenyl)-3-methyl-1-butyl]aminocarbonylmethyl]-benzoic acid as active substance, or of a physiologically acceptable salt thereof, in the preparation of a long-term antidiabetic agent, characterised in that, compared with double the single dose in the administration of a racemate, unnecessarily high and long-lasting substance loading is avoided, as a result of which substantially lower levels of active substance in the plasma are obtained which go beyond the normal advantage of halving the dose in the administration of enantiomers.

**2.** Use of (S)(+)-2-ethoxy-4-[N-[1-(2-piperidinophenyl)-3-methyl-1-butyl]aminocarbonylmethyl]-benzoic acid according to claim 1, characterised in that the active substance with an optical purity of at least ee = 95, or a physiologically acceptable salt thereof, is used.

**3.** Use of (S)(+)-2-ethoxy-4-[N-[1-(2-piperidinophenyl)-3-methyl-1-butyl]aminocarbonylmethyl]-benzoic acid according to claim 1, characterised in that the active substance with an optical purity of at least ee = 98, or a physiologically acceptable salt thereof, is used.

**4.** Use of (S)(+)-2-ethoxy-4-[N-[1-(2-piperidinophenyl)-3-methyl-1-butyl]aminocarbonylmethyl]-benzoic acid as active substance, or a physiologically acceptable salt thereof, in the preparation of a pharmaceutical composition according to claim 1, 2 or 3, characterised in that the single dose is in the range from 0.25 to 5.0 mg.

**5.** Use according to claim 4, characterised in that the single dose is 0.5 mg.

**6.** Use according to claim 4, characterised in that the single dose is 1.0 mg.

**7.** Use according to claim 4, characterised in that the single dose is 2.0 mg.

## Revendications

**1.** Utilisation de l'acide (S) (+)-2-éthoxy-4-[N-[1-(2-pipéridinophényl)-3-méthyl-1-butyl]aminocarbonylméthyl]-benzoïque ou d'un sel physiologiquement acceptable de celui-ci comme principe actif pour la préparation d'un antidiabétique de longue durée caractérisée en ce que, par rapport à la dose unitaire multipliée par deux dans le cas d'une application de racémate, on évite des charges de substance inutilement élevées et de longue durée, de sorte qu'il en résulte des concentrations plasmatiques de principe actif sensiblement plus basses, qui vont au delà de l'avantage normal de la division par deux de la dose dans le cas de l'application d'un énantiomère.

**2.** Utilisation de l'acide (S) (+)-2-éthoxy-4-[N-[1-(2-pipéridinophényl)-3-méthyl-1-butyl]aminocarbonylméthyl]-benzoïque selon la revendication 1 caractérisée en ce que l'on utilise le principe actif avec une pureté optique d'au moins ee = 95 ou un sel physiologiquement acceptable de celui-ci.

**3.** Utilisation de l'acide (S) (+)-2-éthoxy-4-[N-[1-(2-pipéridinophényl)-3-méthyl-1-butyl]aminocarbonylméthyl]-benzoïque selon la revendication 1 caractérisée en ce que l'on utilise le principe actif avec une pureté optique d'au moins ee = 98 ou un sel physiologiquement acceptable de celui-ci.

**4.** Utilisation de l'acide (S) (+)-2-éthoxy-4-[N-[1-(2-pipéridinophényl)-3-méthyl-1-butyl]aminocarbonylméthyl]-benzoïque ou d'un sel physiologiquement acceptable de celui-ci comme principe actif pour la préparation d'un médicament selon la revendication 1, 2 ou 3 caractérisée en ce que la dose unitaire est située dans un domaine de 0,25 à 5,0 mg.

**5.** Utilisation selon la revendication 4 caractérisée en ce que la dose unitaire est de 0,5 mg.

**6.** Utilisation selon la revendication 4 caractérisée en ce que la dose unitaire est de 1,0 mg.

**7.** Utilisation selon la revendication 4 caractérisée en ce que la dose unitaire est de 2,0 mg.

Abbildung 1

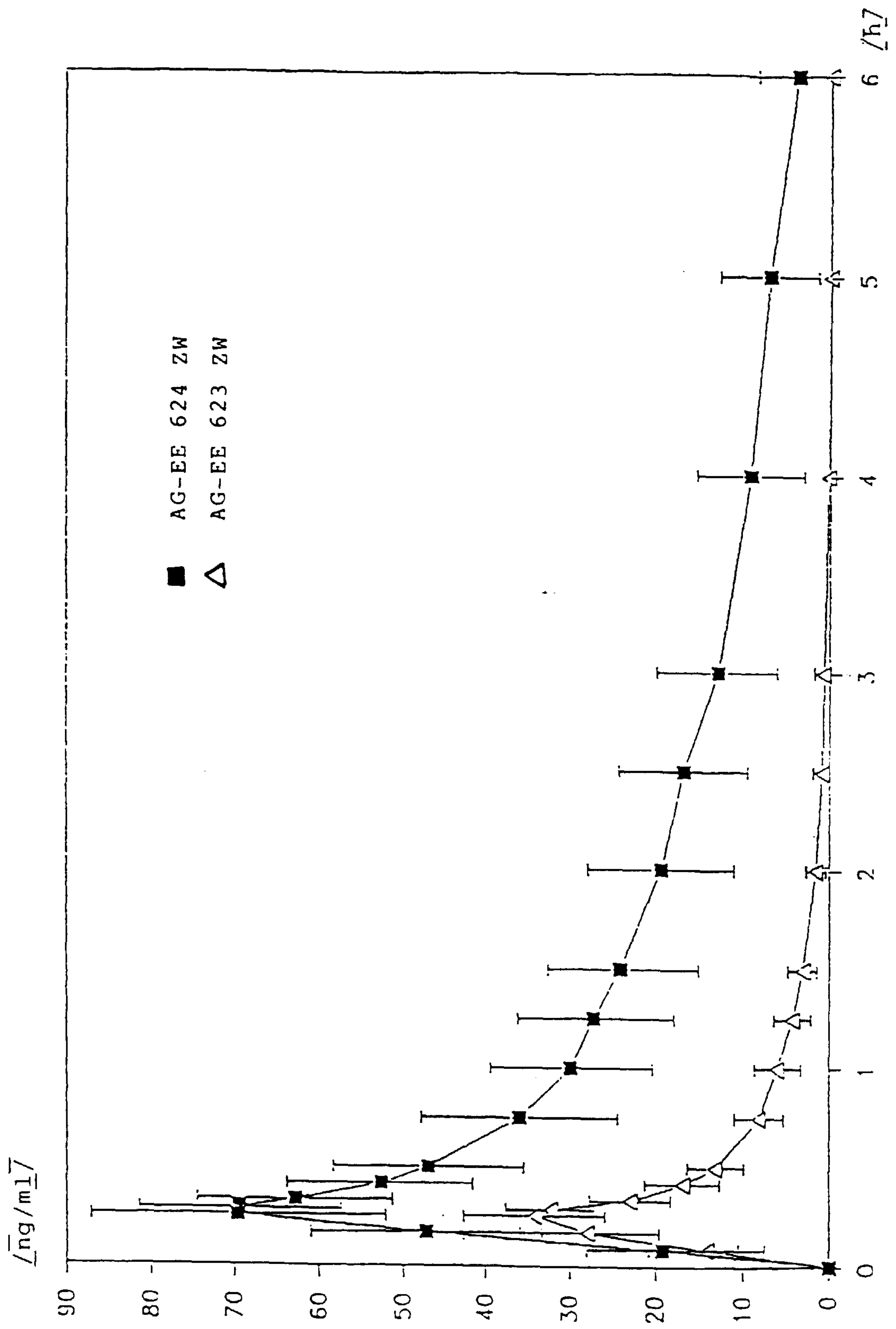

Abbildung 2